# EUROPEAN PATENT APPLICATION

(11) **EP 4 813 350 A1**
(43) Date of publication of application: **30.09.2026**
(21) Application number: 26163881.1
(22) Date of filing: 11.03.2026
(51) Int. Cl.: A61C 17/00, G06T 7/70, G16H 10/60

(54) **TECHNIQUE FOR DEFINING AN ORIENTATION OF A MODEL OF AN ANATOMY OF A PATIENT'S DENTAL ARCH WITHIN A GLOBAL REFERENCE FRAME**

(30) Priority: 24.03.2025 DE 102025111352
(71) Applicant: Institut Straumann AG, 4002 Basel (CH)
(72) Inventor: Winzer, Torben, Berlin (DE)
(74) Representative: Fleuchaus & Gallo Partnerschaft mbB

(57) **Abstract**

A method for defining an orientation of an origin of a model of an anatomy of a patient's dental arch (302; 402) within a global reference frame comprises receiving a dataset comprising a surface mesh, defined by a plurality of polygon, representing the surface of the dental arch (302; 402). For each polygon, a exterior normal vector is determined. A vector sum of exterior normal vectors defines a normal vector, and thereby an orientation, of an occlusion plane (412). A first rotation angle is determined for aligning the normal vector of the occlusion plane (412) with a first direction of the reference frame. An anterior side (504) of the dental arch (302; 402) is defined based on geometric properties of the surface mesh. A second rotation angle is determined for aligning an anterior-posterior direction of the dental arch (302; 402) with a second direction of the reference frame.

## Description

Digital software tools for anatomical treatment planning and/or prosthetic restoration design rely on accurate scan data of anatomical objects of interest together with neighboring anatomical structures such as bone, tissue, nerve tracts, organs and other anatomical structures/objects/features, as well as artificial structures such as surgical implants, abutments, other anchoring systems, artificial analogues of natural structures, among other natural and artificially placed anatomical structures.

In the field of digital dental technology, scan data of anatomical structures are generally determined optically or radiologically. Optical scanners for three-dimensional measurement directly from intra-oral surface structures, or from extra-oral impressions of oral surface structures, are widespread and economical. Usually, surface data is represented by a surface mesh comprising triangular elements, which are routinely saved and exchanged between systems in STL or similar surface definition file formats.

Radiological scanners such as digital volume tomographs (DVT) or computer tomographs (CT) use X-rays to generate volume datasets of anatomical structures. Surface representations can also be determined from these datasets by applying thresholding methods in which the intensity values (measured in Hounsfield units) of the individual scan elements (voxels) are analyzed to determine whether they exceed or fall below certain thresholds. Radiologically dense structures (hereinafter "volumetric density" structures or objects) such as teeth can be identified in this way and the boundary surface of the identified volumetric density scan structures can be modeled as triangulated surface data (e.g. a triangular surface mesh) and again be saved and exchanged between systems in STL or similar surface definition formats.

Computations with magnetic resonance (MR) scan data are more complex, in which contour analysis methods can be applied, which work on the basis of the gradients of adjacent scan elements. However, also in these cases, volumetric structures or objects such as gum, gingiva, and other tissue, predominantly soft tissue, can be identified and its boundary surface modeled as triangulated surface data for further processing.

With the analysis methods described, triangulated surface data of desired surfaces, such as, for example, visible surfaces, or boundary surfaces, such as, for example, inter object surfaces or other invisible surfaces between anatomical objects and/or structures in the scan data can be obtained for all modalities (imaging devices).

Optical surface scan data is typically preferred for the evaluation or computation of virtual tooth restorations due to the ability to achieve high precision without exposing a patient to high-level doses of radiation.

In the context of the present disclosure, the term tooth restoration parts includes every type of object that can be made for the treatment of dental aesthetics and/or defects. Examples are inlays, onlays, partial crowns, crowns, telescopic crowns, bridges, veneers, implant abutments, partial prostheses and prostheses. Also, in the context of the present disclosure, for the sake of simplicity, tooth replacement parts are also covered by the term tooth restoration parts. The term virtual tooth restoration (referred to more briefly as tooth restoration in the following) is to be understood to include appropriate electronic representations of tooth restorations, i.e. digital three-dimensional representations, such as surface representations, of such tooth restoration parts of sufficient accuracy. For example, to manufacture a tooth restoration part, a CAD/CAM data set of the corresponding virtual tooth restoration can be forwarded to a manufacturing machine.

In the context of the present disclosure, scan data can be manipulated and transformed, in some cases predominantly interactively, by use of a graphical user interface, whereby algorithmic assistance is usually used.

Any of the computer-implemented method steps may be directly or indirectly initiated (and/or triggered) by a user input, which may indicate to perform the corresponding step (e.g., for determining any one of the rotation angles and/or translation vectors).

Because of the complex structure of the scan data and the multitude of functional-aesthetic criteria that must be fulfilled-adjustment to opposing dentition, possibly under consideration of jaw movement, adjustment to adjacent teeth under consideration of contact points, adjustment to the preparation lines in order to obtain an optimal margin fit, compliance with minimum material strengths in order to obtain a satisfactory mechanical stability, consideration of desired tooth shapes in the anterior tooth region, etc.-the process of designing a custom tooth restoration, even with the assistance of an interactive computer-aided design tool, conventionally requires a human with the knowledge and experience of both dental restoration design as well as training in using such tools.

To add to the complexity of designing custom prosthetics, prosthetics are often designed to replace an anatomical object that was removed from the existing oral situation. For example, the designer may be tasked with designing a temporary or permanent prosthetic to be placed on an abutment attached to an implant that is placed in a socket of an extracted object. A designer can achieve better accuracy by basing the design of the prosthetic on the patient's actual anatomical contours in the socket left where the extracted object was previously seated. Thus, often, after extraction of the anatomical object, the area had to be optically re-scanned prior to sending the surface model to the designer.

There is an increasing demand for more immediacy in dental treatments. In particular, for procedures that can be completed in one office visit or fewer office visits than would have been possible in the past, novel ways to shorten dental treatment pre-planning are being sought.

In the situation where an anatomical object is targeted for extraction, methodologies are being sought to pre-plan the design of prosthetics such that they can be ready to install in position immediately in place of the extracted anatomical objects in the patient's oral cavity. For example, it may be desired to pre-plan placement of an implant and pre-plan the design of a temporary abutment such that immediately upon tooth extraction (i.e., during the same office visit), an implant can be placed, and a custom temporary abutment installed. It would also be desirable for the abutment to be designed and manufactured, such as, by additive or subtractive manufacturing, e.g. 3D printing or milling, respectively, and installed in the patient's mouth within the same office visit to accommodate immediacy situations as much as possible.

Ideally, a prosthetic should be designed to fit within and conform to the contours of the socket created upon extraction of the anatomical object. However, modern prosthesis design tools such as CAD/CAM systems receive only surface data (represented as a 3D triangular mesh, typically in STL format) without alignment to any three-dimensional (3D) reference frame.

This can lead to poor-fitting or misalignment of the so designed prosthetics when placed into the patent's oral cavity and can in turn lead to patient discomfort, ineffectiveness or other treatment-related troubles.

It is an object of the present disclosure to help alleviate at least some of the disadvantages of known dental restoration or prosthetics design and placement procedures. In particular, it is an object of the present disclosure to help provide a method by which a model of an anatomy of at least a part of a patient's dental arch is oriented within (and/or aligned with) a global reference frame that allows for precise planning of dental treatments and prosthesis design. It is in particular an object to be able to perform the orientation (and/or alignment) for single and dual dental arches as well as full and partial dental arches.

The object is solved by a computer implemented method for defining a position/orientation of an origin and the axes of the origin of a model of an anatomy of at least a part of a patient's dental arch within a global reference frame, by the use of the model with positioned/oriented origin and associated axes for dental treatment planning, such as, object extraction planning, implant planning, artificial dental crown design, and/or prosthesis design, by a computer program product and by a computer-readable storage medium. The dependent represent embodiments of the respective independent claims.

According to a first aspect, a computer-implemented method for defining a position/orientation of an origin and the axes of the origin of a model (that is, a position of the origin and an orientation of the axes of the origin) of an anatomy of at least a part of a patient's dental arch within a global three dimensional-(3D) reference frame, which may also be named generic reference frame, generic coordinate system, global coordinate system or global spatial reference frame in the present disclosure, is provided.

The method comprises a step of receiving a first dataset comprising a first surface mesh representing a surface topology of at least part of the patient's dental arch. The surface topology may comprise at least the crowns of one or multiple teeth, in case the patient still has one or more teeth and/or implants. The first surface mesh is defined by a plurality of polygons, e.g., comprising triangles of different sizes and/or shapes.

The method further comprises a step of determining, for each polygon, a normal vector oriented in an exterior direction of the first surface mesh, that is, away from the side of the dental tissue and towards the patient's oral cavity. The method further comprises a step of determining, such as, by calculating, a main orientation vector defined by a sum over the normal vectors of at least a subset of the polygons defining an orientation of an occlusion plane for the patient's dental arch represented by the first surface mesh. The main orientation vector represents a normal vector of the occlusion plane. The method further comprises a step of determining a first rotation angle for aligning the direction of the main orientation vector with a first main component of the global spatial reference frame.

The method further comprises a step of defining, or identifying, the anterior side of the patient's dental arch based on geometric properties of the first surface mesh.

In an embodiment defining, or identifying, the anterior side of the patient's dental arch based on geometric properties of the first surface mesh may comprise a sub-step of generating a planar projection of at least a selection of the mesh vertices of the first surface mesh into the occlusion plane resulting in a point cloud of the surface mesh vertices in the occlusion plane.

A further sub-step may comprise fitting a segment of a parabola to the generated planar projection. Another further sub-step may comprise defining the anterior side of the patient's dental arch by a position of the vertex of the parabola. This embodiment may in particular be suitable in case the first surface mesh represents a full dental arch, or at least a part of a dental arch that comprises the anterior side of the dental arch.

In another embodiment defining, or identifying, the anterior side of the patient's dental arch based on geometric properties of the first surface mesh, which can be combined with another step for defining, or identifying, the anterior side of the patient's dental arch, may comprise determining an at least approximate orientation of the anterior-posterior vector direction of the patient's dental arch by a weighted sum of at least a subset of the normal vectors. Determining the weighted sum, such as, by calculation, may comprise weighting components of the normal vectors parallel to the occlusion plane higher than components of the normal vectors perpendicular to the occlusion plane. Determining the weighted sum optionally comprises inverting an orientation of the normal vectors directed towards an inside of the patient's dental arch.

The method further comprises a step of determining a second rotation angle for aligning an anterior-posterior vector direction of the patient's dental arch (e.g., an approximate left-right symmetry direction) with a second main component of the global spatial reference plane.

The method further comprises a step of storing an indication of the first rotation angle and of the second rotation angle in association with the first surface mesh.

The method may further comprise a step of providing a second dataset comprising the model of the anatomy of at least the part of the patient's dental arch in the global spatial reference frame. The model may comprise the first surface mesh supplemented with metadata indicative of at least the first rotation angle and the second rotation angle. Optionally, the model may further comprise metadata indicative of one or more translations to align the anatomy of at least part of the patient's arch with the origin of the global spatial reference frame.

By the technique disclosed herein, the model of the anatomy of at least a part of the patient's dental arch is provided in a global spatial reference frame suitable for performing one or more downstream tasks with correct, and/or precise, orientation. The downstream tasks may comprise a virtual dental treatment, such as virtual planning and execution of extracting an object (e.g., a tooth or an implant), virtually planning a prosthesis and/or generating a prosthesis model, and/or virtually setting an implant. For these downstream tasks, it is of great importance that the digital model of the patient's anatomy is correctly and precisely placed and oriented within the global spatial reference frame to allow a multitude of automatic process modules use the digital model.

By providing the correct and precise position/orientation of the origin and its axes of the model, the one or more downstream process steps are thus enabled to be used for performing virtual preparations, treatments, extractions, etc. with improved precision.

The technique disclosed herein may, in particular, be advantageous in cases where the first surface mesh represents only a fraction of the patient's dental arch, e.g., representing only a few teeth, and/or representing missing teeth, such as along only one facial direction.

The first surface mesh comprised in the first dataset may be obtained from, or may be based on, an intraoral scan of the anatomy of the at least part of the patient's dental arch. Alternatively or in addition, the first surface mesh may be obtained from, or may be based on a dental imprint or a model obtained from the dental imprint, such as, for example, a gypsum model.

Any tooth crown as used herein, may be a natural tooth crown or an artificial tooth crown.

The first surface mesh, or any other surface mesh of the disclosure, may comprise smaller polygons in regions of larger curvature, and larger polygons in regions of smaller curvature or flat regions. The polygons may be triangles, rectangles/quadrilaterals, pentagons, hexagons, and/or higher order polygons.

In some embodiments, the first surface mesh comprises crowns of one or more teeth, and optionally at least adjacent regions of the gingiva. In other embodiments, a surface topology of a toothless part of a dental arch, such as, the gingiva surface, may be represented by the first surface mesh. In this case, the particular topology of the gingiva is represented by the first surface mesh.

The exterior of the first surface mesh may comprise a side of the first surface mesh, relative to which a surface envelope appears generally convex. The exterior of the first surface mesh may be oriented away from the one or multiple teeth and/or the gingiva, in particular in case of a toothless part of a dental arch. Alternatively or in addition, the exterior of the first surface mesh may be oriented towards the patient's oral cavity.

Calculating the sum over the normal vectors may correspond to performing the sum, and/or determining an average orientation of the normal vectors.

In some embodiments, the sum over the normal vectors may comprise only a subset of all polygons of the first surface mesh. For example, an extent of the first surface mesh may be determined by a bounding box comprising at least the crowns of the one or multiple teeth. For performing the sum over the normal vectors, only polygons within a subdomain of the bounding box may be used. The subdomain of the bounding box may comprise at least the occlusion surfaces of the crowns. Alternatively or in addition, a subset of the polygons may be selected statistically and/or by tapering off a number of polygon, for example, within a predetermined area or volume of the first surface mesh. Alternatively or in addition, the first surface mesh and/or the bounding box may be cut off close to a boundary between the crowns of one or more teeth and the gingiva. Thereby, normal vectors associated with regions of the gingiva spaced far away from the crowns may be excluded from the summation.

The sum may be simplified by using only a subset of the polygons. Alternatively or in addition, the main orientation vector may be determined with good accuracy if regions far from the crowns of the teeth are excluded. In some embodiments, the sum over the normal vectors may comprise all polygons associated with the crowns of the one or multiple teeth.

The global reference frame may comprise a Cartesian coordinate system, a cylindrical coordinate system, or a spherical coordinate system. Alternatively or in addition, the global reference frame may comprise a predetermined orientation of its main orientation vectors. The Cartesian coordinate system may be spanned by three orthogonal vectors or directions. An X-axis and a Y-axis may span a horizontal XY-plane. A Z-axis may span a vertical direction and be normal to the XY-plane.

The global reference frame may in an example have its vertical, frontal and transversal direction, that is, its Z-direction, Y-direction and X-direction, respectively, relative to a human body's upright position. The X-axis, Y-axis and Z-axis may alternatively be denoted as frontal axis, sagittal axis and vertical axis, respectively; the XY-plane, YZ-plane and XZ-plane may be denoted as transverse plane, sagittal plane and frontal plane, respectively.

Defining a position/orientation of the origin and the axes of the model of the anatomy may comprise performing one or more rotations and/or one or more translations. A first rotation may comprise rotating the occlusion plane to align with the XY-plane of a Cartesian coordinate system, and/or orienting the occlusion plane parallel to the XY-plane of the Cartesian coordinate system. A first translation may comprise translating the occlusion plane after the first rotation to pass through the origin along the vertical direction (Z=0) of the Cartesian coordinate system.

A second rotation may comprise rotating an anterior or posterior portion of the patient's dental arch to align with a predetermined forward direction, e.g., the Y-direction, within the XY-plane. The posterior portion of the patient's dental arch may comprise, or may be located at, a location of the patient's molar teeth (and/or third molars). Alternatively or in addition, the anterior portion of the patient's dental arch may comprise, or may be located at, a location of the patient's incisors.

In any case, the location of the patient's teeth may relate to a conventional anatomical location, even if one or more teeth are absent, for example due to previous extraction, from the patient's dental arch.

A second translation may comprise translating the occlusion plane after the second rotation to pass through the origin of the forward direction (Y=0) of the Cartesian coordinate system. Alternatively or in addition, the second translation may comprise translating the occlusion plane after the second rotation to pass through the origin of a transversal direction (X=0) of the Cartesian coordinate system.

While examples of rotations and/or translations have been described with respect to a Cartesian coordinate system with vertical Z axis, the technique disclosed herein is independent of the choice of global spatial reference frame. Translations and/or rotations can be analogously defined for any of global spatial reference frame and/or 3D coordinate system.

Rotations of the occlusion plane may alternatively be denoted as pitch, yaw and roll. Correspondingly, rotations around the yaw axis may correspond to the second rotation, and/or a rotation within the occlusion plane (and/or a rotation preserving the orientation of the main orientation vector, and/or the normal vector to the occlusion plane). Similarly, rotations around the roll axis and/or around the pitch axis may correspond to the first rotation, and/or a rotation of the occlusion plane, which changes the orientation of its normal vector (and/or the main orientation vector).

In analogy to the use of the terms pitch, yaw and roll, the occlusion plane may correspond to the plane, in which an airplane's wings approximately lie. Rotating the occlusion plane, that is, also its normal vector, to arrive at a horizontal plane of the global reference frame, and, respectively, its normal vector, may correspond to performing a combination of pitch and roll of an airplane. Rotating the occlusion plane around its normal vector may correspond to performing yaw of the airplane.

The first rotation angle may correspond to performing a rotation around a horizontal axis. Correspondingly, the first rotation angle may be denoted as vertical rotation angle, as it serves to rotate the (e.g., direction of the) normal vector of the occlusion plane into a vertical direction. The second rotation angle may correspond to performing a rotation around the vertical axis. Correspondingly, the second rotation angle may be denoted as horizontal rotation angle, as it serves to rotate the occlusion plane within the horizontal plane.

A rotation (and/or rotation angle) may be represented by a transformation matrix (also: rotation matrix). For example, a rotation around the Z axis of a Cartesian coordinate system may be represented by a block-diagonal 3x3 matrix having cosine and (at least partly negative) sine entries of the (in particular first) rotation angle in the upper 2x2 block and 1 as the lower block entry, with rotations around the X or Y axis represented by simultaneous permutations of the columns and rows of the 3x3 matrix. A translation may be represented by (e.g., 3D) translation vector (also: shift vector).

The representation of translations for mapping the origin of the model and the global reference frame may depend on the order, in which rotations and translations are performed. The transformation matrix may be adapted to transposing the first surface mesh into the orientation of the origin with the global spatial reference frame.

Fitting the segment of the parabola to the generated planar projection may comprise fitting parameters of a parabolic function, such as, parameters a, b, and c, of an exemplary parabolic function x = ay² + by +c, to the resulting point cloud of the first surface mesh in the occlusion plane. Fitting the parameters of the parabolic function may comprise minimizing a sum over distances, for example, the quadratic of the distances, of the points in the point cloud from the parabolic function.

The fitted segment of the parabola may be determined as follows. Relative to a first fixed (e.g., orthogonal) two-dimensional (2D) coordinate system within the plane, the parameters of the segment of an optimal parabola are determined by minimizing a sum over the squared distances (or a mean squared distance) of the points in the point cloud relative to the segment of the parabola. The determining of the parameters is repeated for a plurality of other fixed 2D coordinate systems that are each rotated, along the main orientation defined by the main orientation vector of the occlusion plane and/or within the occlusion plane, such as, the Z-axis, by a predetermined angle, for example, 1 degree, relative to the first 2D coordinate system. The best fit of the segment of the parabola corresponds to the optimum among the different fixed coordinate systems.

The fitting of the parameters of the parabolic function may comprise providing the parabolic function in a plurality of 2D coordinate systems at predetermined relative angles within the occlusion plane. The relative angles may correspond to relative angles of the axes from one 2D coordinate system to the next 2D coordinate system within the plurality of 2D coordinate systems. Minimizing the sum over the distances may comprise selecting the 2D coordinate system, in which the sum is smaller than in any of the other coordinate systems.

The indication of the first rotation angle and of the second rotation angle may be stored as metadata associated with the first surface mesh. The metadata may, in addition to the first rotation angle and the second rotation angle, be indicative of one or more translations for aligning the occlusion plane with the origin, such as, for example, at least along the Z-axis and/or the Y-axis, of the global reference frame.

A second dataset may be generated, which comprises the first surface mesh and the metadata in an associative form, that is, one referencing the other or both referencing each other.

The first surface mesh may be obtained by processing an intraoral surface scan of at least part of the patient's oral cavity, and/or of an anatomy of the at least part of the patient's dental arch.

The intraoral surface scan may comprise a scan of at least a part of the patient's dental arch (also: first dental arch), and optionally of at least a part of the patient's opposing dental arch (also: second dental arch), and/or optionally of at least a part of a bite scan in relation to the first dental arch and the second dental arch, in which at least a part of the patient's posterior buccal or lingual jaw portions are scanned, while in an occlusion state. The at least part of the second dental arch may comprise a second occlusion surface opposite to a first occlusion surface of the at least part of the first dental arch.

The first occlusion surface may correspond to the crowns' exposed surface having contact points with the crowns' exposed surface of the second occlusion surface, and vice versa.

In an occlusion state, the first dental arch and the second dental arch of the patient, the first occlusion surface, the second occlusion surface and the occlusion plane may coincide (e.g., at least pointwise at contact points between the first dental arch and the second dental arch) or the first occlusion surface, the second occlusion surface may at least be substantially parallel, that is, their normal vectors pointing in substantially the same direction.

By the processing of the intraoral surface scan, a precise geometric representation of the patient's anatomy of at least the part of the patient's dental arch may be obtained. Thereby, a precision planning of the one or more downstream tasks and/or dental treatments may be facilitated.

The method may comprise a step of obtaining the first surface mesh by clipping a second surface mesh in regions representing the gingiva. The first surface mesh may represent the tooth crowns and at most a height of the gingiva, which is less than a height of the tooth crowns.

The second surface mesh may represent a larger (e.g., fuller) region of the intraoral scan.

By clipping regions of the second surface mesh representing the gingiva, approximately vertically oriented regions of the second surface mesh pertaining to the gingiva at the outer side of the dental arch are clipped, and/or not comprised in the first surface mesh. Alternatively or in addition, regions of the gingiva at the inner side of the dental arch, which approximate horizontal directions of the upper and/or lower jaw, may be clipped, and/or not comprised in the first surface mesh.

A plurality of normal vectors oriented approximately parallel to the occlusion plane at the outer side of the dental arch, and/or a plurality of normal vectors oriented towards a center point of the dental arch and/or away from the occlusion plane, and/or a plurality of vertices far away (in particular displaced by a predetermined minimal distance, which may correspond to a typical height of a crown) from the occlusion plane need not be taken into account or may be removed from the processing. This way, computing resources and/or memory resources can be saved, and/or a computational speed for providing the correctly oriented model can be improved, in particular, without degrading the quality of the correctly oriented model.

The part of the patient's dental arch may comprise one full arch. Determining the anterior side of the patient's dental arch as the position of the vertex of the parabola, a segment of which is fitted to the generated planar projection of mesh vertices, and/or to the resulting point cloud, may particularly be suitable in case of the first surface mesh representing a full arch.

By determining a point cloud, which is projected into the occlusion plane, fitting the segment of the parabola may be reduced to fitting a function of a single variable using a predetermined number of points. The fitted segment of the parabola may essentially run centrally within the point cloud along a length of the dental arch.

By determining the point cloud based on the vertices of the first surface mesh, which essentially only comprises the crowns of one or more teeth, a number of points in the point cloud at an inner boundary and/or at an outer boundary of the dental arch may be reduced compared to determining the point cloud based on the vertices of a second surface mesh, which also represents larger portions of the gingiva. Thereby, a computational speed for fitting the parameters of the parabolic function may be improved. Thus, processing and/or memory resources may be saved.

Fitting the parameters of the parabolic function may comprises providing the parabolic function in a plurality of 2D coordinate systems (in particular rotated relative to the XY-subsystem of the global spatial reference frame) at predetermined relative angles within the occlusion plane. Minimizing the sum over the (in particular quadratic) distances may comprise selecting the 2D coordinate system, in which the sum is smaller than in any of the other coordinate systems.

The predetermined relative angle may be between 1 degree and 5 degrees. The predetermined relative angle may be 1 degree or 2 degrees.

By selecting the parabolic function in the 2D coordinate system with minimal sum over the (in particular quadratic) distances, the fit of the segment of the parabola may be optimized for the first surface mesh comprised in the first dataset.

The part of the patient's dental arch may comprise one full arch. Fitting the segment of the parabola to at least part of the patient's dental arch may comprise determining an approximate orientation of the anterior-posterior vector direction of the patient's dental arch and/or of the anterior direction, e.g., the first horizontal, Y, axis of a Cartesian coordinate system by determining a sum, such as, for example, a weighted sum, over the normal vectors of at least a subset of the normal vectors. Determining the weighted sum may comprise weighting components of the normal vectors parallel to the occlusion plane higher than components of the normal vectors perpendicular to the occlusion plane. Determining the weighted sum optionally comprises inverting an orientation of the normal vectors directed towards an inside of the patient's dental arch.

By orienting the normal vectors towards the outside of the patient's dental arch, a cancellation among components lying within the occlusion plane can be avoided. By performing the weighted sum, an approximate orientation of the Y-axis, and/or a first 2D coordinate system for optimizing parameters of a parabolic function can be determined. Thereby, a number of iterations in determining the parameters of the parabolic function for different orientations of the 2D coordinate system may be minimized, a computational speed improved, and/or processing and/or memory resources may be saved.

Weights may decrease for polygons increasingly displaced from the occlusion plane. By weighting stronger the sum of polygons relatively closer to the occlusion plane, errors due to a diverging shape, e.g., at heights of the gingiva and/or due to missing teeth, can be minimized.

The first dataset may, in particular, in addition to the first surface mesh, comprise at least one designation of a tooth number. The tooth number may be provided according to a reference anatomical model of a dental arch, and/or according to the standard dental notation. The dental notation may be the FDI World Dental Federation (ISO) notation (FDI notation / ISO 3950), the Palmer notation, the universal numbering system, the alphanumeric notation, or the paleoanthropology dental notation.

The designated tooth number can provide information on a position of the first surface mesh along the segment of the parabola. For example, a tooth number corresponding to a molar tooth may indicate a position along an approximately linear segment of the parabola. Alternatively or in addition, a tooth number corresponding to an incisor tooth may be indicative of a position close to the strongest curvature of the parabola. Further alternatively or in addition, a tooth number corresponding to a canine or pre-molar tooth may be indicative of a location on the parabola in a segment transitioning from strong curvature to an approximately vanishing curvature.

The approximately vanishing curvature and/or the approximately linear segment of the parabola need not have a strictly vanishing curvature in the mathematical sense, however, may have less curvature than the frontal teeth.

The knowledge of a tooth number may, in particular, be advantageous in cases of partial or incomplete arches, in which the first surface mesh only represents a smaller number of teeth. The tooth number may be provided for a central tooth represented by the surface mesh. While one tooth number may be sufficient, providing two or more tooth numbers may improve the precision of determining the segment of the parabola.

Fitting the segment of the parabola to at least a part of the patient's dental arch may comprise determining a center of the segment depending on the tooth number and on a target clinical position. The target clinical position may be based on a reference anatomical model (in particular of a full dual dental arch). Thereby, an approximate location along a segment with a predefined range of curvatures can be determined.

The fitting of the segment of the parabola depending on the designated tooth number and on the target clinical position may, in particular, be used for the first surface mesh comprising a partial arch. Using the target clinical position facilitates identifying an expected approximate curvature of the segment of the parabola (e.g., in terms of direction and/or magnitude).

The method may further comprise a step of determining a bounding box comprising the first surface mesh and/or a part of the first surface mesh representing at least a part of a partial arch. For example, in case of a dual arch, two bounding boxes may be determined, one for at least parts of the upper arch, and another one for at least parts of the lower arch. A first face of the bounding box may be selected to minimize a distance from the crowns of the one or multiple teeth. The occlusion plane may be determined to be parallel to the first face of the bounding box.

The bounding box may advantageously be determined after the model of the orientation of the origin and the axes of the model of the anatomy of the at least part of the patient's dental arch has been aligned with the first and second main component of the global spatial reference frame. The faces of the bounding box may in particular each be aligned with a main component of the global spatial reference frame, such as a Cartesian coordinate system.

In an embodiment, the first face of the bounding box may be determined based on the teeth farthest a way from each other, such as the posterior molar teeth and the anterior incisor teeth represented by the first surface mesh.

If the first surface mesh represents a full arch, the bounding boxes often have a length of 50mm or more along the directions perpendicular to the height direction of the crowns. Typically, the bounding box of a first surface mesh has an oblong shape with a ratio f (<length perpendicular to the longest side> to <the length of the longest side>) smaller than 0.65.

It may be determined that the first surface mesh represents a full arch, if the bounding box exceeds a threshold length (e.g., 50mm) along the directions perpendicular to the height direction of the crowns, and/or if the bounding box has an oblong shape with ratio being smaller than a threshold ratio (e.g., smaller than 0.65). Alternatively, it may be determined that the first surface mesh represents a partial arch, if the bounding box does not exceed the threshold length along the directions perpendicular to the height direction of the crowns, and/or if the bounding box does not have an oblong shape with a ratio smaller than the threshold ratio.

Alternative to determining the occlusion plane as a parallel plane to the first face of a bounding box, the method may comprise a step of determining a location of a first face corresponding to a cover plane of the at least part of the dental arch by the tip of a tooth crown of each of a predetermined set of teeth. The predetermined set of teeth may in particular comprise the first incisor and the last molar on one or each side of at least part of the dental arch.

The cover plane may correspond to a plane that essentially covers the at least part of the dental arch by passing through a small number of particularly protruding points. The direction of the protrusion may relate to the patient's oral cavity and/or the exterior of the first surface mesh. For example, a tooth in the lower arch may protrude upwards, and/or a tooth in the upper arch may protrudes downwards.

In some embodiments, the first face corresponding to the cover plane and the first face corresponding to a face of the bounding box may coincide. In other embodiments, the first face corresponding to the cover plane and the first face corresponding to a face of the bounding box may be slightly tilted relative to each other, for example, by an angle smaller than ten degrees.

The first face corresponding to the cover plane can advantageously be determined before or after determining the first rotation angle and/or before or after performing the first rotation. The first face corresponding to the bounding box is advantageously only determined after the occlusion plane has been aligned with the global spatial reference frame and/or after the first rotation has been performed, as typically a bounding box is constructed with faces parallel to the main components of the global spatial reference frame.

The method may further comprise a step of determining a location of the occlusion plane by having a predetermined displacement from the first face, for example, of the bounding box or corresponding to the cover plane, in a direction towards the sockets of the teeth and/or towards the roots of the teeth.

The predetermined displacement may be due to a natural overbite, e.g., of the upper jaw (also: maxilla) over the lower jaw (also: mandible). The occlusion plane may in particular be representative of an average of the occlusion surfaces of the teeth along the dental arch. The average may be displaced relative to the most protruding end of a tooth, such as, for example, an incisor. The predetermined displacement may be in the range of 2mm to 5mm, such as 4mm.

An overbite of a normal denture may be in the range of 2mm to 5mm. A mild abnormal overbite may be in the range of up to approximately 9mm. A severe overbite may be in the range above 9mm.

An indication of the value of the predetermined first displacement may be provided by a user input.

In another embodiment, the at least part of the patient's dental arch comprises at least part of a dual arch. The dual arch may comprise the patient's lower dental arch and the patient's upper dental arch. The method further comprises a step of determining a location of the occlusion plane as an average, such as, an arithmetic mean, of the first face, for example, the bounding box or the cover plane, of the upper dental arch and the first face, for example, the bounding box or the cover plane, of the lower dental arch. The average, such as, the arithmetic mean, may determine the location of the occlusion plane along the vertical axis in a particularly simple manner, and/or without a need to determine a specific displacement value.

The method may further comprise a step of determining a first translation vector (briefly also: first or vertical translation), such as, for example, a vertical translation vector, for translating the occlusion plane to a position at the origin (e.g., Z=0 of the vertical, Z, axis of a Cartesian coordinate system) of the first main component of the global spatial reference frame. The method may further comprise a step of storing a definition of the first translation vector in association with the first surface mesh.

The method may further comprise a step of determining a second translation vector (also: second or horizontal translation) in the occlusion plane to a position of the origin of the occlusion plane at the origin (e.g., X=0, of a second horizontal, X, axis of a Cartesian coordinate system) of a third main component perpendicular to the second main component of the global reference frame.

The second translation vector may be determined such that the anterior side of the patient's dental arch is located at the origin of the third main component. The method may further comprise a step of storing an indication of the second translation vector in association with the first surface mesh.

Determining the second (e.g., horizontal) translation vector may be further based on positioning the origin of the occlusion plane at the origin (e.g., Y=0, of the first horizontal, Y, axis) of the second main component of the global reference frame based on a center-of-mass determination of the patient's dental arch.

The center-of-mass of the segment of the parabola may be located inside the patient's dental arch. Alternatively or in addition, the center of mass may be fictitious, that is, it may correspond to the centroid of a fictitious parabolic area bounded at least in part by the segment of the parabola. The fictious parabolic area may be bounded at its posterior side by a straight line parallel to a connecting line between the first, second or third molars at both sides of the dental arch. Determining the second translation vector may comprise a step of determining a centroid of a parabolic area bounded at least partially by the fitted segment of the parabola. The parabolic area may be bounded in a posterior direction of the at least part of the dental arch by a line parallel to the third main component of the global spatial reference frame (and/or a transversal direction perpendicular to the anterior-posterior vector direction).

The method may further comprise a step of positioning the centroid of the parabolic area at the origin of the second main component of the global spatial reference frame.

Storing, in association with the first surface mesh, the indication of the first translation vector and of the second translation vector may be performed jointly, and in some cases also jointly with storing of the indication of the first rotation angle and of the second rotation angle. The indications of the rotation angles and of the translation vectors may in particular be stored in a second dataset, which comprises the first surface mesh and metadata indicative of the rotation angles and of the translation vectors. Thereby, the model of the anatomy of the at least part of the patient's arch may be provided with the origin oriented within the global spatial reference frame.

According to a second aspect, the orientation of the origin and the axes of the model provided according to the method of the first aspect may be used, amongst other uses, in conjunction with processes for digital dental treatment planning, digital object extraction planning, digital implant planning, digital artificial dental crown design, and/or for digital prosthesis design.

According to a further aspect, a computer program product is provided which, when the program is executed by a computing device, cause the computing device to carry out the steps of the method according to the first aspect.

According to a further aspect, a computer-readable storage medium, including a carrier wave, is provided comprising instructions which, when executed by a computing device, cause the computing device to carry out the steps of the method according to the first aspect.

In all aspects of the present disclosure, using the position/orientation of the origin and the axes of the digital patient model does not require a perfect position/orientation of the occlusion plane, however, a substantially close approximation of a correctly positioned/oriented origin and the axes of the digital patient model is generally sufficient. A sufficiently close precision may, for example, be an accuracy of three degrees of rotation or less from a perfect orientation of the model and/or an accuracy of 3mm or less of translation from a perfect position.

The features of the embodiments of the present disclosure presented hereinabove and hereinbelow can be combined with each other where such combination is considered technically feasible and possibly by the skilled person, unless stated otherwise.

### Brief description of the drawings

Fig. 1 shows an exemplary flowchart of a method for defining a position/orientation of an origin and the axes of a digital model of an anatomy of at least a part of a patient's dental arch within a global spatial reference frame.
Fig. 2 schematically illustrates an architecture of a computing device for defining a position/orientation of an origin and the axes of a model of an anatomy of at least a part of a patient's dental arch within a global spatial reference frame. The computing device may be configured for performing the method.
Fig. 3 shows an exemplary schematic perspective view onto an upper dental arch with a global spatial reference frame indicated, as well as exemplary normal vectors in relation to polygons of a surface mesh representing the upper dental arch.
Fig. 4 shows an exemplary schematic side view onto a dual dental arch comprising both upper and lower dental arch. Bounding boxes per dental arch are indicated as well as a position of the occlusion plane.
Fig. 5 shows an exemplary schematic top view onto a lower dental arch, to which a segment of a parabola is fitted, with the vertex of the parabola determining the anterior direction of the lower dental arch. Tooth numbers are provided for the full dental arch, as an example.
Figs. 6A and 6B schematically illustrate a surface representation of a full dual arch with arbitrary orientation and after alignment of the occlusion plane with a global spatial reference frame, respectively.
Figs. 7A and 7B schematically illustrate a surface representation of a partial dual arch with arbitrary orientation and after alignment of the occlusion plane with a global spatial reference frame, respectively.
Figs. 8A and 8B schematically illustrate a surface representation of a full dual arch with the occlusion plane aligned with the XY-plane, with arbitrary orientation of the Y axis and after alignment of the Y axis with the anterior direction, respectively.
Figs. 9A and 9B schematically illustrate a surface representation of a partial dual arch with the occlusion plane aligned with the XY-plane and with arbitrary position relative to the Y axis and after alignment of the anterior direction with the Y axis, respectively.
Figs. 10A and 10B schematically illustrate a dual dental arch before and after translation of the occlusion plane for alignment with the XY plane at the position Z=0, respectively.
Figs. 11A and 11B show two examples of triangular surface meshes with normal vectors and variable sizes of the triangles depending on a local curvature of the surface, respectively.

### Detailed description

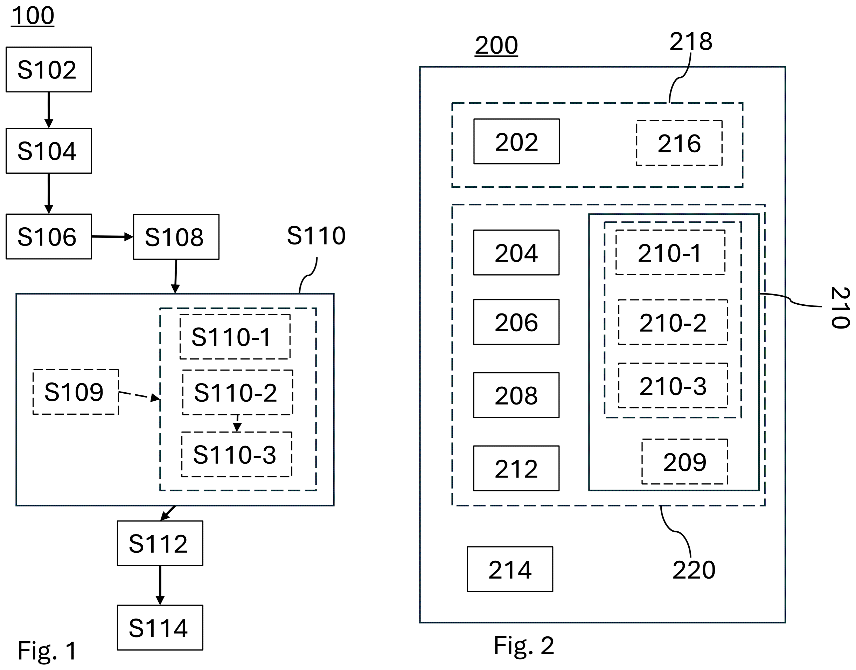

Fig. 1 shows an exemplary flowchart of a computer-implemented method 100 for defining a position/orientation of an origin and the axes of a model of an anatomy of at least a part of a patient's dental arch within a global spatial reference frame

The method 100 comprises a step S102 of receiving a first dataset comprising a first surface mesh representing a surface topology of at least a part of the patient's dental arch (and in some cases comprising at least the crown of one or a plurality of the patient's teeth). The first surface mesh is defined by a plurality of polygons.

The method 100 further comprises a step S104 of determining, for each polygon of the first surface mesh, a normal vector oriented towards an exterior of the first surface mesh. The method 100 further comprises a step S106 of determining a main orientation vector defined by a vector sum of at least a subset of the normal vectors defining an orientation of an occlusion plane for the patient's dental arch represented by the first surface mesh. The main orientation vector represents a normal vector of the occlusion plane.

The method 100 further comprises a step S108 of determining a first rotation angle for aligning the direction of the main orientation vector with a first main component of the global spatial reference frame.

The method 100 may further comprise a step S109 of determining an anterior-posterior vector direction of the patient's dental arch by determining a weighted sum of at least a subset of the normal vectors. Determining the weighted sum may comprise weighting components of the normal vectors parallel to the occlusion plane higher than components of the normal vectors perpendicular to the occlusion plane. Determining the weighted sum optionally comprises inverting an orientation of the normal vectors directed towards an inside of the patient's dental arch.

The method 100 may further comprise a step S110 of defining an anterior side of the patient's dental arch based on geometric properties of the first surface mesh.

The step S110 may comprise a first sub-step S110-1 of generating a planar projection of at least a selection of the mesh vertices of the first surface mesh into the occlusion plane resulting in a point cloud of the surface mesh vertices in the occlusion plane.

The step S110 may further comprise a second sub-step S110-2 of fitting a segment of a parabola to the generated planar projection.

The step S110 may further comprise a third sub-step S110-3 of defining the anterior side of the patient's dental arch by a position of the vertex of the parabola.

The method 100 further comprises a step S112 of determining a second rotation angle for aligning the anterior-posterior vector direction of the patient's dental arch passing through the anterior side with a second main component of the global spatial reference frame. The method 100 still further comprises a step S114 of storing, associated with the first surface mesh, an indication of the first rotation angle and of the second rotation angle. The indication may be provided as metadata in association with the first surface mesh.

Fig. 2 schematically illustrates an architecture of a computing device 200 for defining a position/orientation of an origin and the axes of a model of an anatomy of at least a part of a patient's dental arch within a global spatial reference frame.

The computing device 200 comprises a reception interface 202 configured for receiving a first dataset comprising a first surface mesh representing a surface topology of at least a part of the patient's dental arch (and in some examples comprising at least the crown of one or a plurality of the patient's teeth). The first surface mesh is defined by a plurality of polygons.

The computing device 200 further comprises a first determining unit 204 configured for determining, for each polygon of the first surface mesh, a normal vector oriented towards an exterior of the first surface mesh. The computing device 200 further comprises a second determining unit 206 configured for determining a main orientation vector defined by a vector sum of at least a subset of the normal vectors defining an orientation of an occlusion plane for the patient's dental arch represented by the first surface mesh. The main orientation vector represents a normal vector of the occlusion plane. The computing device 200 further comprises a third determining unit 208 configured for determining a first rotation angle for aligning the direction of the main orientation vector with a first main component of the global spatial reference frame.

The computing device 200 may further comprise a weighted sum performing unit 209, which is configured determining an anterior-posterior vector direction of the patient's dental arch by determining a weighted sum of at least a subset of the normal vectors. Determining the weighted sum may comprise weighting components of the normal vectors parallel to the occlusion plane higher than components of the normal vectors perpendicular to the occlusion plane. Determining the weighted sum optionally comprises inverting an orientation of the normal vectors directed towards an inside of the patient's dental arch.

The computing device 200 may further comprises an anterior side defining unit 210 configured for defining, or identifying, an anterior side of the patient's dental arch based on geometric properties of the first surface mesh.

The anterior side defining unit 210 may comprise a first subunit 210-1 configured for generating a planar projection of at least a selection of the mesh vertices of the first surface mesh into the occlusion plane resulting in a point cloud of the surface mesh vertices in the occlusion plane.

The anterior side defining unit 210 may further comprise a second subunit 210-2 configured for fitting a segment of a parabola to the generated planar projection.

The anterior side defining unit 210 may further comprise a third subunit 210-3 configured for defining the anterior side of the patient's dental arch by a position of the vertex of the parabola. The computing device 200 further comprises a fourth determining unit 212 configured for aligning the anterior-posterior vector direction of the patient's dental arch passing through the anterior side with a second main component of the global spatial reference frame.

The computing device 200 still further comprises a storing unit (also: memory) 214 configured for storing, associated with the first surface mesh, an indication of the first rotation angle and of the second rotation angle. The indication may be provided as metadata in association with the first surface mesh.

The computing device 200 may further comprise a transmission interface 216. The reception interface 202 and the transmission interface 216 may be embodied by an input-output (I/O) interface 218.

The computing device 200 may comprise at least one processor 220. The at least one processor 220 may embody the first determining unit 204, the second determining unit 206, the third determining unit 208, the anterior side defining unit 210, and/or the fourth determining unit 212.

The computing device 200 may be configured for performing the method 100.

The technique disclosed herein (e.g., comprising the method 100 and/or the computing device 200) may be used for automatic dental arch orientation.The technique may be based on a surface mesh representing a dental arch, such as the first and/or second surface meshes.

When entering the acquisition step for obtaining the intraoral surface scan, a rough automated arch orientation may be done, based on heuristic methods. That automated orientation should be done if the case is not precisely enough oriented yet. Therefore, the orientation may be skipped for cases including those that already have been in the acquisition step before, that stem from DW lab scanners, or that would be changed only slightly by the auto-orientation, for example, by less that 1,5 degree rotation and/or less than 1 mm translation, as these cases might be assumed to be already precisely enough positioned/oriented.

The technique disclosed herein (e.g., comprising the method 100 and/or the computing device 200) of auto-positioning and/or auto-orientation can work for all combinations of full and/or partial archs, as well as single and/or dual archs. In particular, the technique disclosed herein are particularly suited for use with semi-edentulous jaw archs, or even fully-edentulous jaw archs.

A distinction between full and partial arches can in some embodiments occur internally.

In case the full arch, such as, an arch covering the molar teeth, also: molars, on both sides as well as the incisor teeth, also: incisors and canines, is represented by a first surface mesh, no further input for automatically positioning/orienting the digital model of the dental arch in the global spatial reference frame may be required.

In case only a partial dental arch is represented by the first surface mesh, tooth designation numbers, possibly without the designated positions, but identifying the teeth present in the partial dental arch, may be required as additional parameter. If no tooth numbers are available, e.g., in a model-only workflow, the orientation of a surface mesh representing a scan of a partial dental arch (also: partial scan) may in some cases be done only partially.

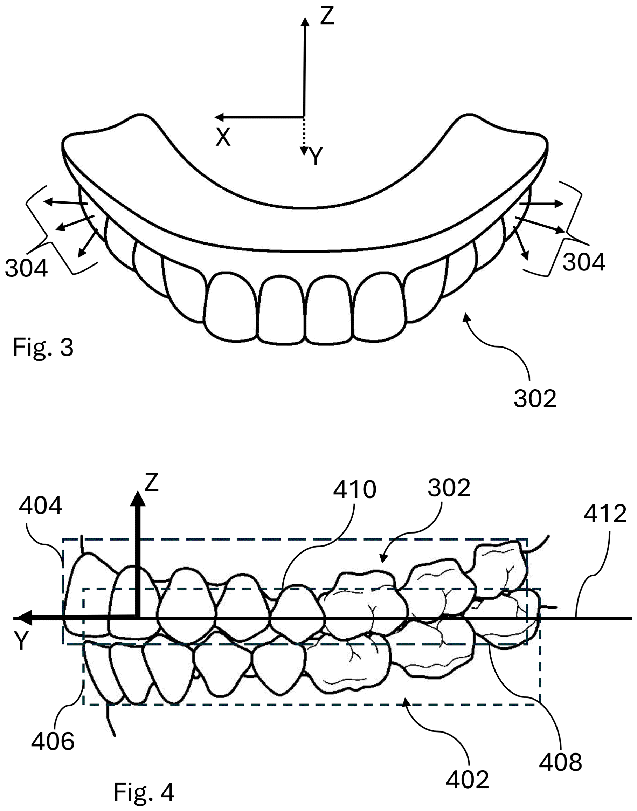

Fig. 3 shows a schematic example of an upper dental arch (also: maxilla) 302 with a global 3D reference frame having the XY-plane parallel to an occlusion plane of the upper dental arch, with the X axis extending laterally (e.g., from the left molars to the right molars) and the Y axis oriented towards the anterior of the dental arch (and/or towards the incisors).

In Fig. 3, exemplarily, a few normal vectors 304 of surface areas represented by different polygons of a first surface mesh representing the upper dental arch 302 are shown. The upper arch 302 in Fig. 3 is an example of a full single arch.

It is noted that the exemplary location of the global spatial reference frame in Fig. 3 is shown with Z=0 at the occlusion plane and Y=0 at an arbitrary position along the anterior direction and/or along the Y axis.

While Figs. 3 to 10B show examples of a Cartesian coordinate system with the Z axis oriented along the vertical direction, the X axis oriented along the transversal direction and the Y axis oriented in the anterior direction of a standing patient, the technique disclosed herein generally applies to different choices of global reference frames.

Fig. 4 shows a schematic side view of a full double arch having upper dental arch (also: maxilla) 302 and lower dental arch (also: mandible) 402. In Fig. 4, the dual dental arch is schematically shown in a closed position, that is, in an occlusal state.

A first bounding box 404 encloses the crowns of the upper dental arch 302, and a second bounding box 406 encloses the crowns of the lower dental arch 402 in Fig. 4. The lower surface 408 of the first (maxilla arch) bounding box 404 is selected to ensure that the occlusion surface of the upper dental arch 302 is just comprised within the first bounding box 404, without extending the first bounding box 404 further downwards. Likewise, the upper surface 410 of the second (mandible arch) bounding box 406 is selected to ensure that the occlusion surface of the lower dental arch 402 is just comprised within the second bounding box 406, without extending the second bounding box 406 further upwards.

The occlusion plane 412 in Fig. 4 is an example of its position for a double arch 302, 402 in which the occlusion plane 412 is determined by the average of the lower surface 408 of the first bounding box 404 and the upper surface 410 of the second bounding box 406. Alternatively, the occlusion surface of each of the mandible and the maxilla could be determined and averaged into a common occlusion surface 412.

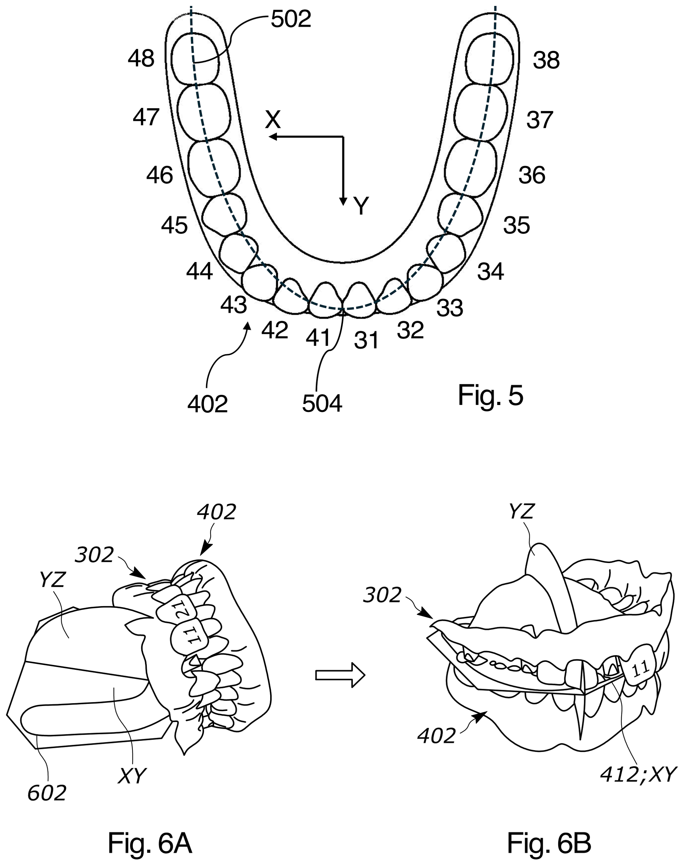

Fig. 5 schematically shows a planar projection of the lower dental arch 402 onto the occlusion plane or a top view onto a lower dental arch 402 with tooth numbers 48, 47, 46, 45,44, 43, 42, 41 for the right side and 31, 32, 33, 34, 35, 36, 37, 38 for the left side provided just outside the dental arch and next to the respective tooth.

Fig. 5 further shows a segment of a parabola 502 fit to the planar projection of the lower dental arch 402, for example, according to sub-step S110-2. At reference sign 504, the vertex of the parabola 502 is indicated, for example, as determined according to the sub-step S110-3. The Y axis is essentially a symmetry axis between left and right sides of the dental arch 402, with the Y axis passing through the vertex 502.

A computation of a rotation matrix may be performed in an automated program, e.g., denoted as cAutomatedArchPreOrientation and classes defined therein.

Any rotation can be represented by a rotation matrix, in particular, including trigonometric functions, such as cosine and sine, of rotation angles as matrix entries, and the total rotation matrix may be given by the product. Any translation can be represented by translation vector.

According to an exemplary embodiment, a first step is to perform an occlusion plane transformation (also: bring the arch into the occlusion plane). This may mean that the dental arch is, or the dental arches are, centered and oriented such that the lower arch occlusion surface is oriented upwards and the upper arch occlusion surface is oriented downwards. The orientation may be performed by aligning an average vertex normal with the Z axis. Upper arch normals may be counted inversely. This step may be performed with a call to the automated program cAutomatedArchPreOrientation.mGetOclusalPlaneTransformation().

Figs. 6A and 6B show a schematic example of a full dual dental arch 302; 402 before and after an occlusion plane transformation, respectively. The occlusion plane transformation may comprise the method steps S104, S106 and S108.

On the left of Fig. 6A, a global reference frame is indicated in terms of its XY plane and its YZ plane, and the anterior region of a scanned dental arch is indicated by the tooth numbers 11, 21 of the upper incisors. In Fig. 6B, an expected position of a full dental arch within the XY plane after rotating and translating the origin of the model of the anatomy of the patient's dental arch is shown.

In Fig. 6B, the occlusion plane 412 is aligned with the XY plane of the global 3D reference frame. However, it is shown how the horizontal directions and/or the direction within the occlusion plane, and/or the anterior side of the dental arch, have not yet been aligned with the global reference frame. The vertical plane 602 is at a random angle and random displacement from the upper incisor 11.

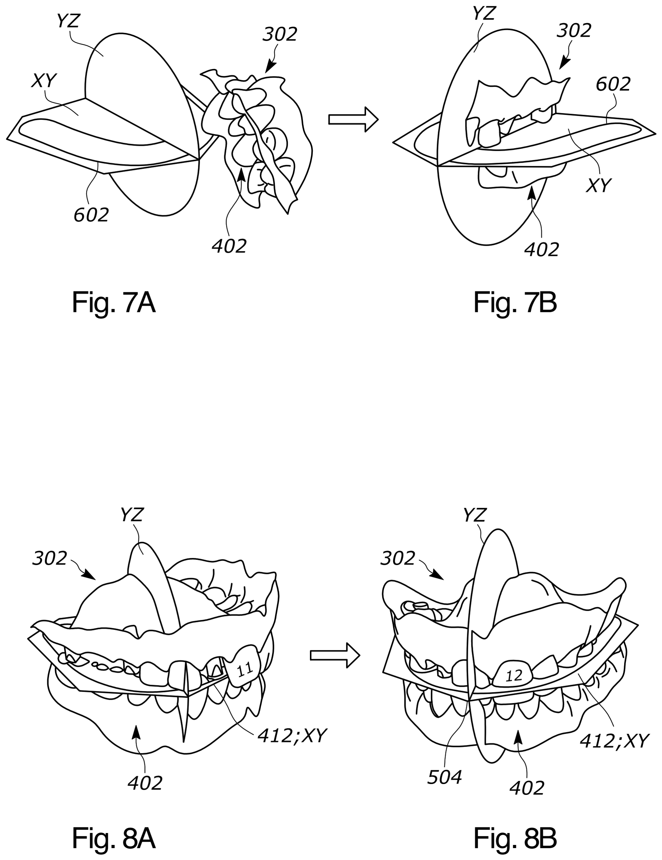

Figs. 7A and 7B show a schematic example of a dual partial arch before and after an occlusion plane transformation, respectively. In Fig. 7A, the occlusion plane is at an arbitrary position and angle relative to the XY plane of the global 3D reference frame.

In Fig. 7A and Fig. 7B, at reference sign 602 an expected position of a full dental arch within the XY plane is shown.

In Fig. 7B, the occlusion plane 412 is aligned, but parallelly displaced upwards, with the XY plane of the global reference frame. The anterior-posterior direction of the dental arch is not yet properly positioned, that is, it needs to be parallelly displaced to act as a symmetry plane of the dual arch, relative to the vertical plane YZ of the global reference frame, as indicated by the expected position of the full dental arch 602, from which the dual partial arch 302; 402 is displaced towards the left of Fig. 7B.

According to an exemplary embodiment, a next step is to find the proper orientation within the occlusion plane. The computation differs here between full arches and partial arches. To check whether the intraoral scan and/or the first surface mesh represent a full arch or a partial arch, the scan and/or a position and/or orientation of the first surface mesh may be transformed to the occlusion plane. The scan and/or the first surface mesh are considered to be representing a full arch, for example, if both sides of a bounding box are larger than 50 mm, and if the shape is oblong, which is defined as (length perpendicular to longest side)/(length longest side)<0.65. Individual computation steps can be performed using different automated programs or subprograms (e.g., denoted as cAutomatedPreOrientationForFullArch for full arch, and/or cAutomatedPreOrientationForPartialArch for partial arch).

For a full dental arch, a goal may be to define, or identify, the anterior side of the dental arch, (e.g., using the automated program cAutomatedPreOrientationForFullArch.mComputeAnteriorVector(). This may be done by summing up surface normal vectors (e.g., per polygon of the surface mesh representing the full dental arch) with inwards pointing normal vectors inverted, normal vectors weighted with the dot product normal*point_coordinates, that gives more weight to normal vectors s pointing radially (and/or horizontally), and/or normal vectors weighted inversely with greater distance from the occlusion plane.

When the vector (or the sum of vectors) pointing to the anterior of the dental arch is found, the orientation of the dental arch within occlusion plane may be given by the matrix and/or angle that aligns the anterior vector with the Y axis.

Fig. 8A and 8B schematically show a full dual arch before and after the transformation within the occlusion plane, respectively. In Fig. 8A, the anterior side of the dental arch is not aligned with the vertical plane YZ of the global reference frame, as visible from the incisor with tooth number 11. In Fig. 8B, the anterior side of the dental arch is substantially aligned with the Y axis, as shown by the position of the YZ plane and the incisor 12.

For the placement within the occlusion plane for partial arches, the tooth number may be needed or at least advantageously known in an embodiment. From the tooth number and the target clinical position, the following can be computed: a central and/or average tooth number, a position of the average tooth number according to the target clinical position, and the bow direction, and/or tangent at the position. For example, the automated program cAutomatedPreOrientationForPartialArch.mGetAverageAssignationinformation() can be used in this case. Assuming that the scan is done symmetrically around a tooth having a specified tooth number, the scan (or the first surface mesh representing it) can be centered at the computed averaged tooth position.

As a next step, the longest side of the arch can be aligned with an arch tangent, e.g., according to an automated subprogram mComputeOcclusionRotation.

Finally, the bending of the arch may be determined, or identified, to decide if the desired orientation is already obtained, or if a rotation by 180° is needed (such as to flip between upper arch and lower arch orientations), e.g., according to an automated subprogram mlsPiFlipNeeded. To determine, or identify, the dental arch direction, the two furthest points of the scan or the scans may be computed, e.g., according to an automated subprogram mFindFarestPoint. Considering these two points as the chord of the arch, a center-of-mass and/or bary center of the arch lies on the inner side of the chord.

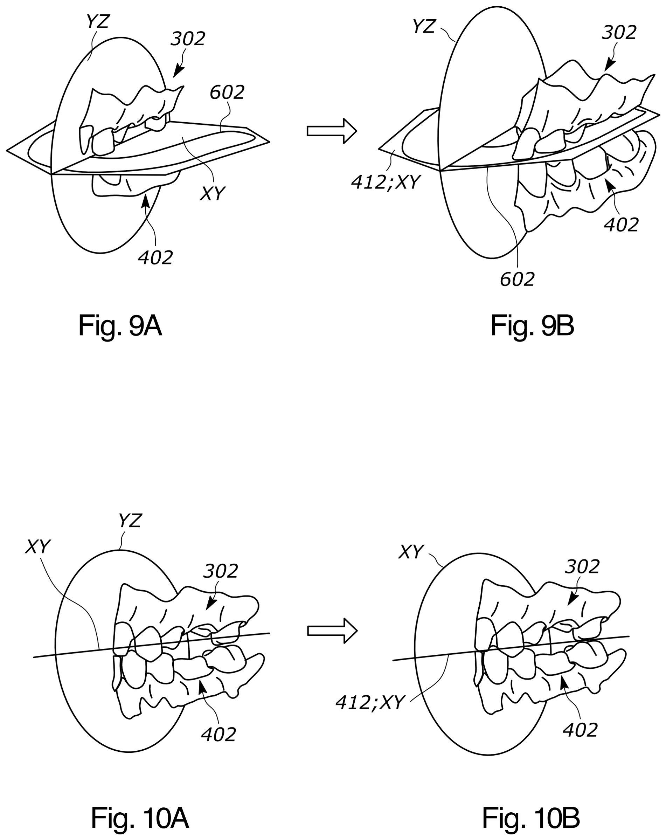

Fig. 9A and 9B schematically illustrate a position of a partial dual dental arch 302; 402 before and after translation and/or before and after a second translation vector is applied within the occlusion plane, respectively.

In Fig. 9A, the expected position of the full dental arch at reference sign 602 is displaced from the partial dental arch 302; 402 before the translation, and the occlusion plane is displaced from the XY plane. In Fig. 9B, the partial dental arch 302; 402 is placed at the expected position 602 of the full dental arch after the translation, and the occlusion plane 412 lies within the XY plane.

The positioning in Z direction may at first simply have been performed by centering. This may give a bad result for the Z position of the occlusion. A final Z alignment can improve the occlusion positioning. For the case of a dual arch, the average of the lowest point of the upper arch and the highest point of the lower arch may be aligned with the Z=0 level in the example of a Cartesian coordinate system.

Fig. 10A and 10B schematically illustrate a dual arch before and after occlusion alignment, respectively. In Fig. 10A, the occlusion plane (not shown) lies below the XY plane of the Cartesian coordinate system before a translation and/or before applying a first translation vector. In Fig. 10B, the occlusion plane 412 coincides with the XY plane after translation.

For the case of a single arch, the arch may be moved such that the occlusion overlaps, e.g., 4 mm, with the Z=0 level, e.g., according to an automated subprogram cAutomatedArchPreOrientation.OCCLUSION_OVERLAP.

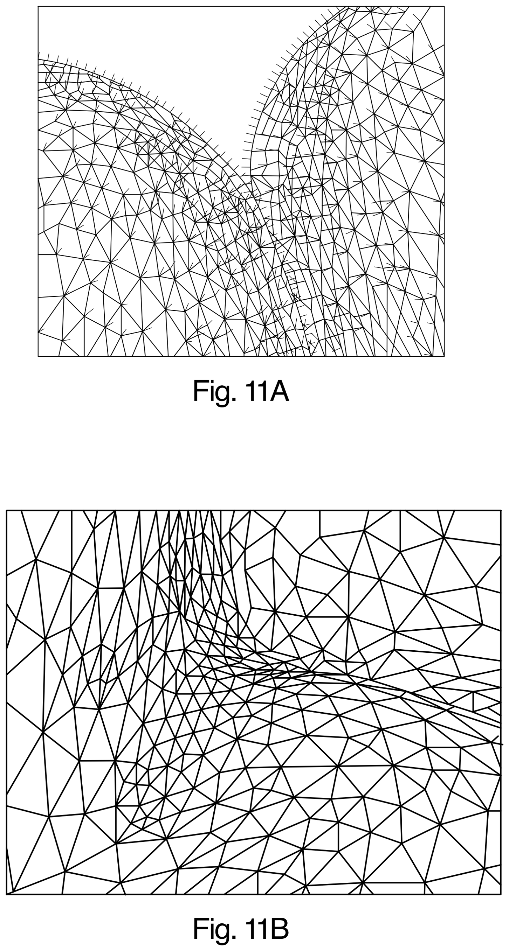

Fig. 11A shows a first example of a triangular surface mesh with normal vectors. In Fig. 11A, instead of surface normal vectors, vertex normal vectors are depicted.

Vertex normal vectors can be determined by averaging the surface normal vectors of polygons directly surrounding the vertex. Optionally, the averaging of the surface normal vectors can comprise weighting the surface normal vector of each polygon by a function of the area (e.g., by a normalized area) enclosed by the polygon. For a smoother varying approximation, surface normal vectors of polygons further away from the vertex can in principle also be taken into account. However, this smoother varying approximation may be less accurate.

In the example of Fig. 11A, the triangular surface mesh is only shown along tooth crowns. No surface mesh is shown along the adjacent gingiva.

Fig. 11B shows a further example of a triangular surface mesh with smaller triangles in regions of larger curvature of the surface and larger triangles in regions of smaller curvature of the surface.

A large curvature, such as, at an approximate right angle can occur for example between a tooth and an adjacent area, where a tooth is missing. A further example of a region of large curvature is example the posterior end of the dental arch, such as from the last molar towards the gingiva in direction of the jaw joint.

## Claims

1. Computer-implemented method (100) for defining an orientation of an origin and axes of a model of an anatomy of at least a part of a patient's dental arch (302; 402) within a global spatial reference frame, comprising the steps of:
- Receiving (S102) a first dataset comprising a first surface mesh representing a surface topology of at least a part of the patient's dental arch (302; 402), wherein the first surface mesh is defined by a plurality of polygons;
- Determining (S104), for each polygon of the first surface mesh, a normal vector (304) oriented towards an exterior of the first surface mesh;
- Determining (S106) a main orientation vector defined by a vector sum of at least a subset of the normal vectors (304) defining an orientation of an occlusion plane (412) for the patient's dental arch (302; 402) represented by the first surface mesh, wherein the main orientation vector represents a normal vector of the occlusion plane (412);
- Determining (S108) a first rotation angle for aligning the direction of the main orientation vector with a first main component of the global spatial reference frame;
- Defining (S110) an anterior side (504) of the patient's dental arch (302; 402) based on geometric properties of the first surface mesh;
- Determining (S112) a second rotation angle for aligning an anterior-posterior vector direction of the patient's dental arch (302; 402) passing through the anterior side (504) with a second main component of the global spatial reference frame; and
- Storing (S114), in association with the first surface mesh, an indication of the first rotation angle and of the second rotation angle.

2. The method (100) of claim 1, wherein the first surface mesh is obtained from an intraoral surface scan of the anatomy of the at least part of the patient's dental arch (302; 402).

3. The method (100) of claim 1 or 2, wherein defining (S110) the anterior side (504) of the patient's dental arch (302; 402) based on geometric properties of the first surface mesh comprises:
- Generating (S110-1) a planar projection of at least a selection of the mesh vertices of the first surface mesh into the occlusion plane (412) resulting in a point cloud of the surface mesh vertices in the occlusion plane (412);
- Fitting (S110-2) a segment of a parabola (502) to the generated (S110-1) planar projection; and
- Defining (S110-3) the anterior side (504) of the patient's dental arch (302; 402) by a position of the vertex (504) of the parabola (502).

4. The method (100) of any one of claims 1 to 3, wherein the polygons are selected from a group comprising triangles, rectangles, pentagons, hexagons, and/or higher order polygons.

5. The method (100) of claim 4, wherein the first surface mesh comprises a plurality of polygons of different sizes, and/or with different numbers of vertices.

6. The method (100) of any one of claims 1 to 5, wherein the first rotation angle and/or the second rotation angle is represented by a transformation matrix, which is adapted to transposing the first dataset into the orientation of the origin within the global spatial reference frame.

7. The method (100) of any one of claims 1 to 6 in combination with claim 3, wherein fitting (S110-2) a segment of a parabola (502) to the generated (S110-1) planar projection comprises fitting the parameters of a parabolic function to the point cloud, wherein fitting the parameters of the parabolic function comprises minimizing a sum over, in particular quadratic, distances of the points in the point cloud from the parabolic function.

8. The method (100) of claim 7, wherein fitting the parameters of the parabolic function comprises providing the parabolic function in a plurality of two-dimensional, 2D, coordinate systems at predetermined relative angles to each other within the occlusion plane (412), and wherein minimizing the sum over the, in particular quadratic, distances comprises selecting the two-dimensional coordinate system, in which the sum is smaller than in any of the other coordinate systems.

9. The method (100) of any one of claims 1 to 8, wherein defining (S110) an anterior side (504) of the patient's dental arch (302; 402) based on geometric properties of the first surface mesh comprises determining (S109) an approximate orientation of the anterior-posterior vector direction of the patient's dental arch (302; 402) by determining a weighted sum of at least a subset of the normal vectors (304), wherein determining the weighted sum comprises weighting components of the normal vectors (304) parallel to the occlusion plane (412) higher than components of the normal vectors (304) perpendicular to the occlusion plane (412), wherein determining the weighted sum optionally comprises inverting an orientation of the normal vectors (304) directed towards an inside of the patient's dental arch (302; 402).

10. The method (100) of claim 9, wherein the weights are further decreasing for polygons increasingly displaced from the occlusion plane (412).

11. The method (100) of any one of claims 1 to 10, wherein the first dataset further comprises a designation of a tooth number in respective positions of the tooth.

12. The method (100) of claim 11 in combination with claim 3, wherein fitting (S110-2) a segment of a parabola (502) to the generated (S110-1) planar projection comprises determining a center of the segment of the parabola (502) to be fit (S110-2) depending on the tooth number and on a target clinical position.

13. The method (100) of any one of claims 1 to 12, further comprising the step of determining a bounding box (404; 406) comprising the first surface mesh, wherein a first face (408; 410) of the bounding box (404; 406) is selected to minimize a distance from the crowns of the one or multiple teeth, and wherein the occlusion plane (412) is determined to be parallel to the first face (408; 410) of the bounding box (404; 406).

14. The method (100) of any one of claims 1 to 12, further comprising the step of determining a location of a first face corresponding to a cover plane of the at least part of the dental arch by the tip of a tooth crown of each of a predetermined set of teeth, wherein the predetermined set of teeth in particular comprises the first incisor and the last molar on one or each side of the at least part of the dental arch.

15. The method (100) of claim 13 or 14, wherein the at least part of the patient's dental arch (302; 402) comprises at least part of a single arch, wherein a single arch comprises the patient's lower dental arch ( 402) or the patient's upper dental arch (302), wherein the method (100) further comprises the steps of:
- Determining a location of the occlusion plane (412) by having a predetermined displacement from the first face (408; 410) in direction towards the sockets of the teeth and/or towards the roots of the teeth;
- Determining a first translation vector for translating the occlusion plane (412) to a position at the origin of the first main component of the global spatial reference frame; and
- Storing the first translation vector in association with the first surface mesh.

16. The method (100) of claim 8, wherein the predetermined displacement is in the range of 2mm to 5mm, preferably 4mm.

17. The method (100) of claim 6 or 7, wherein the at least part of the patient's dental arch (302; 402) comprises at least part of a dual arch, wherein the dual arch comprises the patient's lower dental arch (402) and the patient's upper dental arch (302), wherein the method (100) further comprises the steps of:
- Determining a location of the occlusion plane (412) as an average, in particular an arithmetic mean, of the first face (408) of the upper dental arch (302) and the first face (410) of the lower dental arch (402);
- Determining a first translation vector for translating the occlusion plane (412) to a position at the origin of the first main component of the global spatial reference frame; and
- Storing the first translation vector in association with the first surface mesh.

18. The method (100) of any one of claims 1 to 1 to 17, further comprising the steps of:
- Determining a second translation vector in the occlusion plane (412) to a position of the origin of the occlusion plane (412) at the origin of a third main component perpendicular to the second main component of the global spatial reference frame, such that the anterior side (504) of the patient's dental arch is located at the origin of the third main component; and
- Storing the second translation vector in association with the first surface mesh.

19. The method (100) of claim 18 in combination with claim 3, wherein determining the second translation vector further comprises:
- Determining a centroid of a parabolic area bounded at least partially by the fitted (S110-2) segment of the parabola (502); and
- Positioning the centroid of the parabolic area at the origin of the second main component of the global spatial reference frame.

20. Use of the model oriented within the global reference frame according to the method of any one of claims 1 to 19, for dental treatment planning, object extraction planning, implant planning, artificial dental crown design, and/or prosthesis design.

21. A computer program product which, when the program is executed by a computing device (200), cause the computing device (200) to carry out the steps of the method (100) according to any one of claims 1 to 19.

22. A computer-readable storage medium comprising instructions which, when executed by a computing device (200), cause the computing device (200) to carry out the steps of the method (100) according to any one of claims 1 to 19.
